# EUROPÄISCHE PATENTANMELDUNG

(11) **EP 1 930 033 A2**
(43) Veröffentlichungstag der Anmeldung: **11.06.2008**
(21) Anmeldenummer: 07450224.6
(22) Anmeldetag: 05.12.2007
(51) Int. Cl.: A61M 1/02

(54) **System für die Kryokonservierung von Körperflüssigkeiten und Verfahren zur Verwendung eines derartigen Systems**

(30) Priorität: 05.12.2006 AT 20152006
(71) Anmelder: Beer, Franz, Dr., 3001 Mauerbach (AT); Ruckser, Reinhard, Dr., 1220 Wien (AT)
(72) Erfinder: Beer, Franz, Dr., 3001 Mauerbach (AT); Ruckser, Reinhard, Dr., 1220 Wien (AT)
(74) Vertreter: Nemec, Harald

(57) **Zusammenfassung**

Ein System (1) zur Kryokonservierung von Nabelschnurblut zum Anschluss an ein Fraktionierungssystem (2) zur Fraktionierung der Blutfraktionen wird angegeben. Das Fraktionierungssystem (2) weist zumindest einen Plasmabeutel (3) und zumindest einen Buffy-Coat-Beutel (4) mit Stammzellenkonzentrat sowie absperrbare Verbindungen zwischen den Komponenten auf. Das System (1) zur Kryokonservierung umfasst zwei durch einen Verbindungsschlauch (6) über ein Drei-Wege-Ventil (9) miteinander verbundene Spritzen (7, 8), wobei an einem ersten Anschluss (10) des Drei-Wege-Ventils (9) eine erste Spritze (8) angeschlossen ist und an einem zweiten Anschluss (11) des Drei-Wege-Ventils (9) eine zweite Spritze (7) angeschlossen ist. An einem dritten Anschluss (12) des Drei-Wege-Ventils (9) ist ein Anschlussschlauch (13) zur Ausbildung einer sterilen Verbindung des Systems (1) zur Kryokonservierung mit dem Fraktionierungssystem (2) angeschlossen.

## Beschreibung

Die Erfindung betrifft ein System für die Kryokonservierung von Nabelschnurblut zum Anschluss an ein Fraktionierungssystem zur Fraktionierung der Blutfraktionen, wobei das Fraktionierungssystem zumindest einen Plasmabeutel und zumindest einen Buffy-Coat-Beutel mit Stammzellenkonzentrat sowie absperrbare Verbindungen zwischen den Komponenten aufweist, und wobei das System zur Kryokonservierung zwei durch einen Verbindungsschlauch über ein Drei-Wege-Ventil miteinander verbundene Spritzen umfasst, wobei an einem ersten Anschluss des Drei-Wege-Ventils eine erste Spritze angeschlossen ist und an einem zweiten Anschluss des Drei-Wege-Ventils eine zweite Spritze angeschlossen ist, sowie ein Verfahren zur Verwendung eines derartigen Systems.

Aus der DE 101 51 343 A1 ist ein Beutelsystem für die Kryokonservierung von Körperflüssigkeiten wie Blut, Knochenmark oder Nabelschnurblut bekannt. Die Teile des Beutelsystems sind sterilisiert und voneinander abtrennbar. Flüssigkeiten, die eine Kryokonservierung ermöglichen und/oder unterstützen, befinden sich im Beutelsystem und/oder lassen sich in das Beutelsystem steril einbringen. Das Beutelsystem umfasst eine Vorrichtung zur direkten Entnahme der Körperflüssigkeit aus dem lebenden Körper, einen oder mehrere Bereiche zum Mischen der Flüssigkeiten mit der Körperflüssigkeit und/oder zur Einlagerung des Gemisches aus Flüssigkeiten und Körperflüssigkeit, mehrere Absperrorgane und Verbindungsleitungen und ein Einlassorgan und/oder ein Auslassorgan. Das Beutelsystem ist dabei so ausgelegt, dass die Teile des Beutelsystems, die durch die Verbindungsleitungen miteinander kommunizieren, vor dem Einsatz ein geschlossenes steriles System bilden und dass die Bereiche zur Einlagerung des Gemisches aus Flüssigkeiten und Körperflüssigkeit voneinander hermetisch verschließbar und abtrennbar sind.

Das in der DE 101 51 343 A1 beschriebene System ist jedoch zur nicht zur sterilen Umsetzung, Vermischung und Lagerung konzipiert, sondern vielmehr zur Separation von einzelnen Zellen aus einer Flüssigkeit, also dem entgegengesetzten Prozess.

Weiterhin ist aus der WO 1992/07243 eine Vorrichtung und ein Verfahren zur Trennung von Partikeln mittels eines biegsamen Gefäßes bekannt. Die dort beschriebene Vorrichtung weist ein Schlauchsystem und zwei mittels eines Schlauchstücks mit einer Klemme miteinander verbundene Spritzen aufweist, wobei ein Drei-Wege-Ventil direkt mit dem Schlauchstück, mit einer der Spritzen und mit dem Separationsbeutel verbunden ist.

Nachteilig ist bei diesem bekannten Stand der Technik vor allem, dass das Verfahren eine Vielzahl von Komponenten und Verfahrensschritten benötigt und daher aufwendig durchzuführen ist.

Es ist demnach Aufgabe der Erfindung, ein System und ein Verfahren zur Kryokonservierung von Körperflüssigkeiten anzugeben, welche zuverlässige Sterilität des Systems gewährleisten, einfach in der Handhabung bzw. Durchführung und kostengünstig in der Herstellung sind.

Die Aufgabe wird gelöst durch ein System zur Kryokonservierung, welches sich dadurch auszeichnet, dass an einem dritten Anschluss eines Drei-Wege-Ventils ein Anschlussschlauch zur Ausbildung einer sterilen Verbindung des Systems zur Kryokonservierung mit dem Fraktionierungssystem angeschlossen ist.

Weitere vorteilhafte Ausgestaltungsformen der Erfindung sind aus den Unteransprüchen ersichtlich.

Die sterile Verbindung ist vorteilhafterweise durch ein Sterilschlauch-Schweißverfahren herstellbar, welches zuverlässig und einfach ist.

Vorteilhafterweise ist das System hermetisch abgeschlossen, wodurch aufwendige Reinraumprozeduren vermieden werden können.

In der ersten Spritze ist bevorzugt ein Kryopreservans bevorratet, welches somit vorteilhafterweise bereits steril vorliegt und nicht extra in das System eingebracht werden muss.

Als vorteilhaft hat sich das Kryopreservans Dimethylsulfoxid erwiesen, welches auch ungekühlt aufbewahrt werden kann und somit bereits in der Sterilverpackung vorliegen kann.

An dem die erste Spritze mit dem Drei-Wege-Ventil verbindenden Verbindungsschlauch ist eine lösbare Klemmeinrichtung vorgesehen ist, durch welche vorteilhafterweise ein vorzeitiger Übertritt des Kryopreservanss verhinderbar ist.

In dem System ist mittels der zweiten Spritze durch Aufziehen der Spritze ein Vakuum herstellbar, so dass vorteilhafterweise keine weitere Verbindung mit einer Evakuierungseinheit erforderlich ist, um die Stammzellenlösung zur Lagerung fertigzustellen.

Besonders vorteilhaft ist, wenn das System als steril verpacktes Einweg-System ausgebildet ist, da dies die Lagerung und die Anwendung vereinfacht. Die einzelnen Komponenten sind sowohl kostengünstig in der Herstellung als auch in der Sterilverpackung, so dass sie nach der Verwendung entsorgt werden können, ohne aufwendige Reinigungsmaßnahmen zu erfordern.

Ein bevorzugtes Ausführungsbeispiel der Erfindung ist in den Figuren schematisch dargestellt und in der nachfolgenden Beschreibung detailliert erläutert. In den Figuren zeigen:
- Fig. 1: eine stark schematisierte Darstellung eines Fraktionierungssystems, zum Anschluss an welches das erfindungsgemäß ausgestaltete System geeignet ist, und
- Fig. 2: eine schematische Darstellung eines erfindungsgemäß ausgestalteten Systems zur Kryokonservierung von Körperflüssigkeiten.

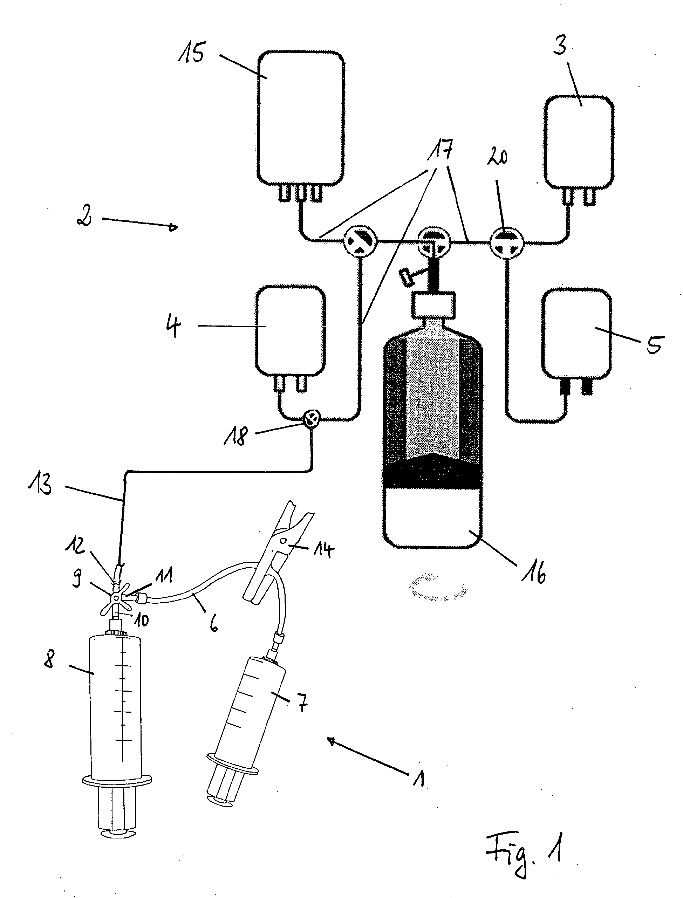

In Fig. 1 ist in einer stark schematisierten Ansicht ein an sich bekanntes Ausführungsbeispiel eines Fraktionierungssystems 2 zur Separation der Bestandteile von Körperflüssigkeiten wie beispielsweise Blut dargestellt. Da das Fraktionierungssystem 2 an sich bekannt ist, wird hier nur kurz auf die zur Erläuterung der Erfindung nötigen Bestandteile des Fraktionierungssystems 2 eingegangen. Da das weiter unten näher beschriebene erfindungsgemäß ausgestaltete System 1 zur Kryokonservierung von Körperflüssigkeiten neben weiteren Anwendungen insbesondere zur Kryokonservierung von aus Nabelschnurblut gewonnenem Stammzellenkonzentrat geeignet ist, wird die Erfindung unter Bezugnahme auf diesen speziellen Anwendungsfall diskutiert.

Humane Nabelschnurstammzellen finden seit ca. zehn Jahren therapeutische Anwendung bei der Behandlung verschiedener Erkrankungen. Vor dem Hintergrund stetig wachsender Indikationsgebiete (z.B. Blutstammzell-Rescue bei der zytostatischen Behandlung bösartiger Krankheiten, Tumor-Vaccinationen, immunologischer Therapieansätze, regenerative Medizin) gewinnt die Langzeitlagerung von Nabelschnurstammzellen im Sinne von Eigenvorsorge oder für immunologisch passende Verwandte des Spenders zunehmend Bedeutung. Das beschriebene System 1 ist dafür geeignet, jedoch nicht darauf beschränkt.

Das Fraktionierungssystem 2 umfasst in bekannter Weise im Wesentlichen eine Zentrifuge 16, mittels welcher die Bestandteile beispielsweise von Nabelschnurblut, welches aus der Nabelschnur eines Neugeborenen zur Stammzellenkonservierung entnommen wurde, separiert werden können. Das Nabelschnurblut befindet sich zunächst in einem Nabelschnurblutbeutel 15, wird der Zentrifuge 16 zugeführt und während verschiedener Verfahrensschritte in die Bestandteile rote Blutkörperchen ((Erythrozyten), Blutplasma und Stammzellenkonzentrat (Buffy-Coat) separiert. Die verschiedenen Bestandteile werden über Verbindungen 17 in jeweils zumindest einen Blutbeutel 5, Plasmabeutel 3 und Buffy-Coat-Beutel 4 geleitet.

In dem Buffy-Coat-Beutel 4 liegt nach Abschluss der Separation das Stammzellenkonzentrat vor, welches nun konserviert werden muss. An die zwischen dem Buffy-Coat-Beutel 4 und der Zentrifuge 16 ausgebildete Verbindung 17 ist das erfindungsgemäß ausgestaltete System 1 über einen geeigneten Anschluss 18, z.B. ein Drei-Wege-Ventil 9, angeschlossen.

Das übliche Konservierungsverfahren für das Stammzellenkonzentrat erfolgt mittels Kryokonservierung, also durch Einkühlen des Buffy Coats mittels flüssigen Stickstoffs zur Langzeitlagerung. Um zu vermeiden, dass Verunreinigungen des Stammzellenkonzentrats auftreten, müssen diverse Verfahrensschritte wie beispielsweise Umfüllen oder Beimengen von Kryopreservans unter sterilen Bedingungen in einem Reinraum und/oder in einem hermetisch abgeschlossenen System stattfinden.

Die Herstellung von Reinstraumbedingungen ist stets ein äußerst kostspieliges und aufwendiges Unterfangen, welches einen hohen Einsatz von finanziellen, personellen und räumlichen Ressourcen erfordert. Um diesen Aufwand zu reduzieren, ist erfindungsgemäß vorgesehen, die vorbereitenden Schritte zur Kryokonservierung des Stammzellenkonzentrats in einem hermetisch abgeschlossenen System 1 durchzuführen, welches aus wenigen, einfach zu handhabenden und kostengünstigen Bestandteilen besteht und jederzeit auch außerhalb eines Reinraums verwendbar ist. Die Komponenten sind dabei so kostengünstig, dass das System nach der Verwendung nicht aufwendig und teuer gereinigt werden muss, sondern entsorgt werden kann.

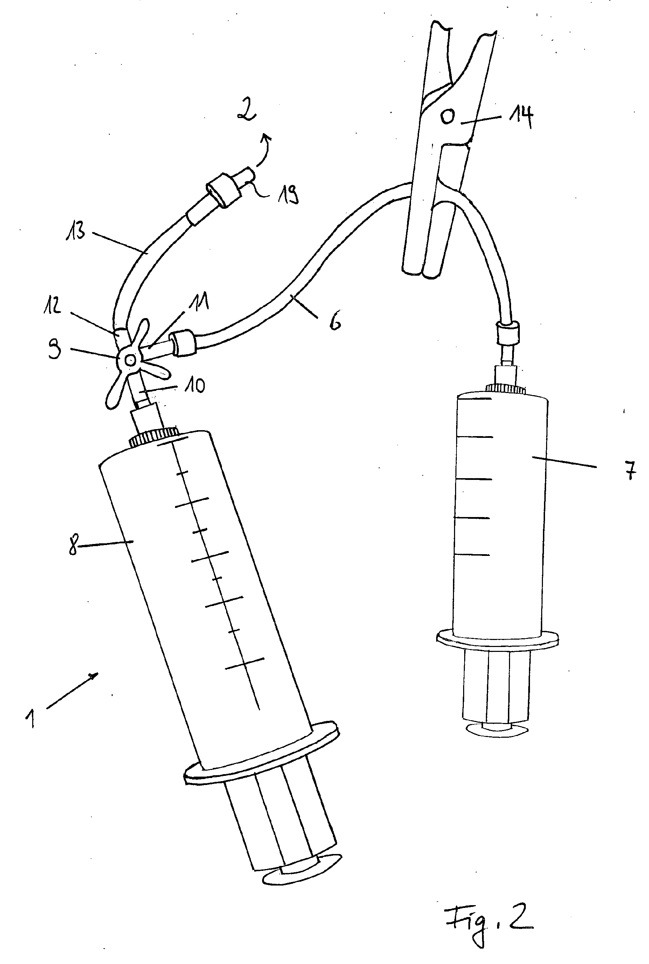

Ein bevorzugtes Ausführungsbeispiel eines erfindungsgemäß ausgestalteten Systems 1 ist in Fig. 2 in einer stark schematisierten Darstellung gezeigt.

Das System 1 zur Kryokonservierung ist dabei vorzugsweise als steril verpacktes Spritzenensemble konzipiert und umfasst eine erste Spritze 8, eine über einen Verbindungsschlauch 6 mit dieser verbundene zweite Spritze 7 und einen Anschlussschlauch 13, über welchen die Verbindung an das Fraktionierungssystem 2 erfolgt. Die Verbindung zwischen den Spritzen 7 und 8 sowie mit dem Fraktionierungssystem 2 erfolgt über ein Drei-Wege-Ventil 9, welches über drei Anschlüsse 10, 11 und 12 verfügt. Am ersten Anschluss 10 setzt die erste Spritze 8 an, am zweiten Anschluss 11 ist der Verbindungsschlauch 6 zur zweiten Spritze 7 angeordnet, und am dritten Anschluss 12 befindet sich der Anschlussschlauch 13 zum Fraktionierungssystem 2. Die zweite Spritze 7 beinhaltet bereits in der Sterilverpackung das dem Buffy Coat zuzumischende Kryopreservans (vorzugsweise Dimethysulfoxid, DMSO) sowie eine Klemmvorrichtung 14 auf dem Verbindungsschlauch 6 zwischen den beiden Spritzen 7 und 8 zur Vermeidung des zu frühen Übertretens des DMSO in die erste Spritze 8. Insbesondere soll durch die Klemmvorrichtung 14 vermieden werden, dass das Kryopreservans, welches Lösungsmitteleigenschaften hat, dauerhaft an dem Drei-Wege-Ventil 9 anliegt, welches dadurch möglicherweise beschädigt würde. Wird das Drei-Wege-Ventil 9 nur kurzzeitig von Kryopreservans bzw. einem das Kryopreservans enthaltenden Gemisch durchströmt, ist jedoch keine Beschädigung des Drei-Wege-Ventils 9 zu befürchten. Die Klemmvorrichtung 14 kann wie dargestellt eine Klemmschere sein, jedoch sind auch weitere an sich bekannte Klemmvorrichtungen wie Klammern oder auch das Zurückbiegen des Verbindungsschlauchs 6 auf sich selbst mit einer entsprechenden Fixierung möglich.

Die aseptische Behandlung des Stammzellenkonzentrats ist integrale Forderung bei der Lagerung, da eine Verunreinigung durch infektiöse Mikroorganismen die Verwendbarkeit der Stammzellen entweder wesentlich erschwert oder oftmals ganz unmöglich macht.

Daher wird erfindungsgemäß vorgeschlagen, die Übergabe des Buffy Coat aus dem Buffy-Coat-Beutel 4 des Fraktionierungssystems 2 sowie die Zugabe von Kryopreservans zum Buffy Coat durch das sterile Spritzenensemble des Systems 1 unter Zuhilfenahme einer Sterilverschweißung des Spritzenensembles des Systems 1 mit dem Fraktionierungssystem durchzuführen. Ein freies Schlauchende 19 des Anschlussschlauch 13 des Spritzenensembles des Systems 1 ist bei Auslieferung des Systems 1 verschlossen, so dass die Sterilität des Systems 1 gewährleistet ist. Mittels an sich bekannter Sterilschlauch-Schweißtechnik wird so nach der Entnahme des Systems 1 aus der Sterilverpackung eine sterile Verbindung zwischen dem Fraktionierungssystem 2 und dem System 1 zur Kryokonservierung hergestellt, die die Hermetizität des System 1 gewährleistet. Weitere Verbindungen zu anderen Systemkomponenten können über beliebige weitere Verzweigungsventile in gleicher Weise vorgenommen werden, ohne die hermetische Abgeschlossenheit und somit die Sterilität zu beeinträchtigen.

Im Folgenden wird das Verfahren zur Kryokonservierung von Nabelschnurstammzellenkonzentrat mittels des erfindungsgemäß ausgestalteten Systems 1 zur Kryokonservierung beschrieben.

Grundsätzlich erfolgt die Kryokonservierung über die folgenden drei Verfahrensschritte, die jeweils wiederum mehrere Aufgaben umfassen: Mischen von Plasma mit Kryopreservans, Mischen von Plasma-Kryopreservans-Gemisch mit dem Inhalt des zumindest einen Buffy-Coat-Beutels, und Kryopreservierung des Gemisches aus Plasma, Kryopreservans und Buffy Coat.

Im ersten Verfahrensschritt werden zunächst das System 1 und das Fraktionierungssystem 2 bei geschlossenem Drei-Wege-Ventil 9 des Systems 1 miteinander steril verschweißt. Sodann wird das Drei-Wege-Ventil 9 so verdreht, dass die zweite Spritze 7 mit dem Fraktionierungssystem 2 in Verbindung steht. Mittels der zweiten Spritze 7 wird Luft zum Anziehen von Spenderplasma aus dem Fraktionierungssystem 2 aus dem Plasmabeutel 3 angesaugt. Der Plasmabeutel 3 wird sodann vom System 1 durch einen Absperrhahn 20 getrennt. Danach wird das Drei-Wege-Ventil 9 so gestellt, dass eine Verbindung zwischen dem Fraktionierungssystem 2 und der ersten Spritze 8 hergestellt wird und die Zufuhr einer definierten Menge von Spenderplasma in die erste Spritze 8 erfolgen kann. Das Plasma wird zu dem in der ersten Spritze 8 bevorrateten DMSO gemischt. Im nächsten Schritt erfolgt das Absaugen von überschüssiger Luft aus der ersten Spritze 8 mittels der zweiten Spritze 7.

Nun wird das Drei-Wege-Ventil 9 zur Verbindung der zweiten Spritze 7 mit dem Fraktionierungssystem 2 umgestellt, um eine Abgabe der abgesaugten Luft in das Fraktionierungssystem 2 bzw. in den Plasmabeutel 3 zu ermöglichen. Zumindest ein Buffy-Coat-Beutel 4 ist entweder bereits an das Fraktionierungssystem 2 angeschlossen und mit diesem verbunden, oder dieser wird an das Fraktionierungssystem 2 angeschlossen und mit diesem verbunden und zusammen mit der ersten Spritze 8 auf eine Temperatur von ca. 4°C eingekühlt. Ein erneutes Verstellen des Drei-Wege-Ventils 9 zur Verbindung der ersten Spritze 8 mit dem Fraktionierungssystem 2 ermöglicht das Absaugen von Luft mittels der zweiten Spritze 7 aus dem Buffy-Coat-Beutel 4. Nun kann eine Vermischung des Plasma-Kryopreservans-Gemischs mit dem Inhalt des zumindest einen Buffy-Coat-Beutels 4 in dem zumindest einem Buffy-Coat-Beutel 4 erfolgen. Abschließend wird in diesem Verfahrensschritt der zumindest eine Buffy-Coat-Beutel 4 in bereits beschriebener Weise entlüftet.

Der dritte, das Verfahren zur Kryokonservierung abschließende Verfahrensschritt sieht vor, dass zumindest ein Kryo-Beutel an den zumindest einen Buffy-Coat-Beutel 4 steril angeschweißt wird, das Gemisch aus Plasma, Kryopreservans und Buffy Coat aus dem zumindest einen Buffy-Coat-Beutel 4 in den zumindest einen Kryo-Beutel erfolgt und dieser dann steril vorzugsweise mittels einer PVC-Schweißzange verschweißt und abgetrennt wird. Die Gemischmenge beträgt dabei üblicherweise 20ml pro Beutel. Alternativ kann auch die Verwendung eines Kryo-Beutels entfallen und statt dessen der zumindest eine Buffy-Coat-Beutel 4 selbst steril verschweißt, abgetrennt und eingefroren werden.

Weiterhin ist es möglich, das so behandelte Gemisch zusätzlich einem Verfahrensschritt der Qualitätskontrolle durch Entnahme einer definierten Quantität des Gemisches aus dem zumindest einen Buffy-Coat-Beutel zu unterziehen, um sicherzustellen, dass das Gemisch von einwandfreier steriler Qualität ist.

Das Verfahren ist sowohl einfach als auch zügig durchführbar, so dass der Buffy Coat schnell verarbeitet werden kann und nicht unnötig lange bei zu hohen Temperaturen verweilt. Eine aufwendige Bereitstellung des Reinraumes ist genauso wenig nötig wie die Einschulung des Personals auf Reinraumnutzung.

## Patentansprüche

1. System (1) zur Kryokonservierung von Nabelschnurblut zum Anschluss an ein Fraktionierungssystem (2) zur Fraktionierung der Blutfraktionen, wobei das Fraktionierungssystem (2) zumindest einen Plasmabeutel (3) und zumindest einen Buffy-Coat-Beutel (4) mit Stammzellenkonzentrat sowie absperrbare Verbindungen zwischen den Komponenten aufweist, und wobei das System (1) zur Kryokonservierung zwei durch einen Verbindungsschlauch (6) über ein Drei-Wege-Ventil (9) miteinander verbundene Spritzen (7, 8) umfasst, wobei an einem ersten Anschluss (10) des Drei-Wege-Ventils (9) eine erste Spritze (8) angeschlossen ist und an einem zweiten Anschluss (11) des Drei-Wege-Ventils (9) eine zweite Spritze (7) angeschlossen ist, **dadurch gekennzeichnet, dass** an einem dritten Anschluss (12) des Drei-Wege-Ventils (9) ein Anschlussschlauch (13) zur Ausbildung einer sterilen Verbindung des Systems (1) zur Kryokonservierung mit dem Fraktionierungssystem (2) angeschlossen ist.

2. System nach Anspruch 1, **dadurch gekennzeichnet, dass** die sterile Verbindung durch ein Sterilschlauch-Schweißverfahren herstellbar ist.

3. System nach Anspruch 1 oder 2, **dadurch gekennzeichnet, dass** das System (1) hermetisch abgeschlossen ist.

4. System nach einem der Ansprüche 1 bis 3, **dadurch gekennzeichnet, dass** in der ersten Spritze (8) ein Kryopreservans bevorratet ist.

5. System nach Anspruch 4, **dadurch gekennzeichnet, dass** das Kryopreservans Dimethylsulfoxid ist.

6. System nach einem der Ansprüche 1 bis 4, **dadurch gekennzeichnet, dass** an dem die erste Spritze (8) mit dem Drei-Wege-Ventil (9) verbindenden Verbindungsschlauch (6) eine lösbare Klemmeinrichtung (14) vorgesehen ist, durch welche ein vorzeitiger Übertritt des Kryopreservanss verhinderbar ist.

7. System nach einem der Ansprüche 1 bis 6, **dadurch gekennzeichnet, dass** in dem System (1) mittels der zweiten Spritze (7) durch Aufziehen der Spritze (7) ein Vakuum herstellbar ist.

8. System nach einem der Ansprüche 1 bis 7, **dadurch gekennzeichnet, dass** das System (1) als steril verpacktes Einweg-System (1) ausgebildet ist.

9. Verwendung eines Systems (1) gemäß einem der vorstehenden Ansprüche zur Kryokonservierung von Nabelschnurstammzellen.

10. Verfahren zur Kryokonservierung von Nabelschnurblut mittels eines Systems (1) zur Kryokonservierung von Nabelschnurblut geeignet zum Anschluss an ein Fraktionierungssystem (2) zur Fraktionierung der Blutfraktionen, wobei das Fraktionierungssystem (2) zumindest einen Plasmabeutel (3) und zumindest einen Buffy-Coat-Beutel (4) mit Stammzellenkonzentrat sowie absperrbare Verbindungen zwischen den Komponenten aufweist, und wobei das System (1) zur Kryokonservierung zwei durch einen Verbindungsschlauch (6) über ein Drei-Wege-Ventil (9) miteinander verbundene Spritzen (7, 8) umfasst, wobei an einem ersten Anschluss (10) des Drei-Wege-Ventils (9) eine erste Spritze (8) angeschlossen ist und an einem zweiten Anschluss (11) des Drei-Wege-Ventils (9) eine zweite Spritze (7) angeschlossen ist, und wobei an einem dritten Anschluss (12) des Drei-Wege-Ventils (9) ein Anschlussschlauch (13) zur Ausbildung einer sterilen Verbindung des Systems (1) zur Kryokonservierung mit dem Fraktionierungssystem (2) angeschlossen ist, **dadurch gekennzeichnet, dass** das Verfahren folgende Verfahrensschritte umfasst:
- Mischen von Plasma mit Kryopreservans,
- Mischen von Plasma-Kryopreservans-Gemisch mit dem Inhalt des zumindest einen Buffy-Coat-Beutels (4), und
- Kryopreservierung des Gemisches aus Plasma, Kryopreservans und Buffy Coat.

11. Verfahren nach Anspruch 10, **dadurch gekennzeichnet, dass** der erste Verfahrensschritt folgende Teilschritte umfasst:
- Steriles Verbinden des Systems (1) mit dem Fraktionierungssystem (2) bei geschlossenem Drei-Wege-Ventil (9),
- Verstellen des Drei-Wege-Ventils (9) zur Verbindung der zweiten Spritze (7) mit dem Fraktionierungssystem (2),
- Absaugen von Luft mittels der zweiten Spritze (7) zum Anziehen von Spenderplasma aus dem Fraktionierungssystem (2),
- Verstellen des Drei-Wege-Ventils (9) zur Verbindung des Fraktionierungssystems (2) mit der ersten Spritze (8),
- Zuführen einer definierten Menge von Spenderplasma in die erste Spritze (8),
- Zumischen von Plasma zu einem in der ersten Spritze (8) bevorrateten Kryopreservans, und
- Absaugen von überschüssiger Luft aus der ersten Spritze (8) mittels der zweiten Spritze (7).

12. Verfahren nach Anspruch 11, **dadurch gekennzeichnet, dass** das Kryopreservans Dimethylsulfoxid ist.

13. Verfahren nach Anspruch 10, **dadurch gekennzeichnet, dass** der zweite Verfahrensschritt folgende Teilschritte umfasst:
- Verstellen des Drei-Wege-Ventils (9) zur Verbindung der zweiten Spritze (7) mit dem Fraktionierungssystem (2),
- Abgabe der abgesaugten Luft in das Fraktionierungssystem (2),
- gegebenenfalls Anschließen und/oder Verbinden zumindest eines Buffy-Coat-Beutels (4) an das Fraktionierungssystem (2),
- Kühlen des zumindest einen Buffy-Coat-Beutels (4) und der ersten Spritze (8),
- Verstellen des Drei-Wege-Ventils (9) zur Verbindung der ersten Spritze (8) mit dem Fraktionierungssystem (2),
- Absaugen von Luft mittels der zweiten Spritze (7) aus dem zumindest einen Buffy-Coat-Beutel (4),
- Mischen des Plasma-Kryopreservans-Gemischs mit dem Inhalt des zumindest einen Buffy-Coat-Beutels (4) in dem zumindest einem Buffy-Coat-Beutel (4), und
- Entlüften des zumindest einen Buffy-Coat-Beutels (4).

14. Verfahren nach Anspruch 13, **dadurch gekennzeichnet, dass** der zumindest eine Buffy-Coat-Beutel (4) und die erste Spritze (8) auf ca. 4°C gekühlt werden.

15. Verfahren nach Anspruch 10, **dadurch gekennzeichnet, dass** der dritte Verfahrensschritt folgende Teilschritte umfasst:
- Steriles Anschweißen zumindest eines Kryo-Beutels an den zumindest einen Buffy-Coat-Beutel (4),
- Umfüllen des Gemisches aus Plasma, Kryopreservans und Buffy Coat aus dem zumindest einen Buffy-Coat-Beutel (4) in den zumindest einen Kryo-Beutel, und
- Steriles Verschweißen und Abtrennen des zumindest einen Kryo-Beutels.

16. Verfahren nach Anspruch 10, **dadurch gekennzeichnet, dass** der dritte Verfahrensschritt alternativ die folgenden Teilschritte umfasst:
- Steriles Verschweißen und Abtrennen des zumindest einen Buffy-Coat-Beutels (4), und
- Einfrieren des zumindest einen Buffy-Coat-Beutels (4).

17. Verfahren nach einem der Ansprüche 10 bis 16, **dadurch gekennzeichnet, dass** das Verfahren zusätzlich den Verfahrensschritt einer Qualitätskontrolle durch Entnahme einer definierten Quantität des Gemisches aus dem zumindest einen Buffy-Coat-Beutel (4) umfasst.

18. Verfahren nach einem der Ansprüche 10 bis 17, **dadurch gekennzeichnet, dass** das Verbinden der Buffy-Coat- und Kryo-Beutel mittels eines Sterilschlauch-Schweißverfahrens erfolgt.

19. Verfahren nach einem der Ansprüche 10 bis 16, **dadurch gekennzeichnet, dass** das sterile Verschweißen und Abtrennen der Buffy-Coat- und Kryo-Beutel mittels einer PVC-Schweißzange erfolgt.
